Europäisches Patentamt

European Patent Office

Office européen des brevets

(11) Publication number: **0 424 531 A1**

(12) **EUROPEAN PATENT APPLICATION**
published in accordance with Art.
158(3) EPC

(21) Application number: 89909047.6

(22) Date of filing: 04.08.89

(86) International application number:
PCT/JP89/00803

(87) International publication number:
WO 90/01474 (22.02.90 90/05)

(51) Int. Cl.⁵: **C07C 19/08**, C07C 17/20,
B01J 27/10, C07B 61/00

(30) Priority: 05.08.88 JP 194596/88

(43) Date of publication of application:
02.05.91 Bulletin 91/18

(84) Designated Contracting States:
DE FR GB IT NL

(71) Applicant: ASAHI GLASS COMPANY LTD.
1-2, Marunouchi 2-chome
Chiyoda-ku Tokyo 100(JP)

(72) Inventor: YONEDA, Hajime
542-2, Kikuma Ichihara-shi
Chiba-ken 290(JP)
Inventor: TAKEI, Ruytaro
1846-1, Goi Ichihara-shi
Chiba-ken 290(JP)

(74) Representative: Wächtershäuser, Günter, Dr.
Tal 29
W-8000 München 2(DE)

(54) **PROCESS FOR PRODUCING 1,1-DICHLORO-2,2,2-TRIFLUOROETHANE.**

(57) The invention relates to a process for producing 1,1-dichloro-2,2,2-trifluoroethane, which comprises reacting 1,1,2-trichloro-2,2-difluoroethane (R122) and/or 1,1,2,2-tetrachloro-1-fluoroethane (R121) with HF in a liquid phase in the presence of antimony pentachloride. This process can depress production of an isomer, R123a, as a by-product.

EP 0 424 531 A1

## PROCESS FOR PRODUCING 1,1-DICHLORO-2,2,2-TRIFLUOROETHANE

### TECHNICAL FIELD

The present invention relates to a process for producing 1,1-dichloro-2,2,2-trifluoroethane.

### BACKGROUND ART

1,1-dichloro-2,2,2-trifluoroethane (hereinafter referred to as R123) is useful by itself as a foaming agent, as a solvent or as a cooling medium. Further, it can be converted to $CHClFCF_3$ (R124) which is useful as an operating fluid, by fluorinating it with anhydrous hydrogen fluoride. It is also known that trifluoroacetic acid can be produced by its photo-oxidation and hydrolysis. Thus, it is a useful compound for industrial purposes.

Heretofore, it is known that $CHCl_2CF_3$ (R123) can be obtained by fluorinating tetrachloroethylene with anhydrous hydrogen fluoride in a gas phase in the presence of a fluorinating catalyst [J.Fluorine Chem., 13 (1979) 7-18]. However, the yield is extremely low, and such a method can not be regarded as an economically feasible industrial method. Besides, R123 obtained by the fluorination in the gas phase, contains from 10 to 15% of isomer 1,2-dichloro-1,2,2-trifluoethane ($CF_2ClCHFCl$, R123a), whereby a step of isomerizing or removing such isomer, is required.

### PROBLEM TO BE SOLVED BY THE PRESENT INVENTION

The object of the present invention is to solve the above problem inherent to the conventional method for the production of R123.

### MEANS TO SOLVE THE PROBLEM

The present invention has been made to solve the above problem and provides anew a process for producing 1,1-dichloro-2,2,2-trifluoroethane (R123), which comprises reacting 1,1,2-trichloro-2,2-difluoroethane (R122) and/or 1,1,2,2-tetrachloro-1-fluoroetane (R121) with HF in a liquid phase in the presence of antimony pentachloride.

In the present invention, R121 and/or R122 may be used as the starting material. R121 or R122 can be obtained by the fluorination of trichloroethylene. However, it can be obtained also by the liquid phase fluorination of tetrachloroethylene. For example, in a case where tetrachloroethylene is used, in the first stage of reaction, R121 can be formed by the addition reaction of tetrachloroethylene and HF, and then R122 can be formed by the fluorination of this R121. Whereas, in a case where trichloroethylene is used, pentachloroethane is formed by a chlorine-addition reaction, and then R121 or R122 can be formed by the fluorination reaction thereof.

In the present invention, R121 and/or R122 is reacted with HF in a liquid phase, as mentioned above. The fluorination from R121 to R122 and the fluorination from R122 to R123, proceed successively. Therefore, these reactions are preferably conducted as a continuous series of reactions.

If the amount of HF to be reacted is less than the stoichiometric amount, it is difficult to efficiently obtain R123. On the other hand, if the amount is excessive, the production of R123 tends to decrease, such being undesirable. Therefore, HF is preferably used in an amount of from 1 to 3 mols per mol of R122 and from 2 to 6 mols per mol of R121.

It is known that antimony pentachloride as the catalyst is partially fluorinated in the presence of HF to form $SbCl_xF_y$ ($x+y=5$, $0<x<5$, $0<y<5$), and it is believed that the fluorinated product functions as the catalyst. Accordingly, it is preferred to supply HF prior to or during the reaction in an amount sufficient for the fluorination of antimony pentachloride. If the amount of the catalyst is too small, the effects of the catalyst will be small. On the other hand, if the amount is too much, the catalyst will be wasted, such being economically undesirable. Therefore, the amount of the catalyst is usually preferably from 2 to 100 mol% relative to the compound to be fluorinated.

The higher the reaction temperature, the better, since the reaction rate of the compound to be fluorinated thereby improves. However, if the temperature is too high, there will be a problem such that the selectivity decreases, or decomposition of the catalyst takes place. Therefore, the reaction temperature is preferably from 50 to 150 °C, although it depends also on the reaction pressure. The higher the reaction pressure, the higher the reaction rate and the production rate. However, if the pressure is too high, there will be a problem that the apparatus must have a pressure durable structure. Therefore, the reaction is preferably conducted within a range of from 0 to 25 kg/cm²G. Further, in order to prevent the decomposition of the catalyst, the reaction may be conducted in the presence of $Cl_2$.

### EXAMPLES

EXAMPLE 1

Into a 4 ℓ stainless steel pressure container, 4.5 kg of antimony pentachloride was charged, and 3 kg of HF was fed at 60°C under 0 K/G for fluorination. While heating the mixture at 130°C, HF was supplied at a rate of 360 g/hr and R122 was supplied at a rate of 3.1 kg/hr. The formed gas was passed through a sodium hydroxide cleaning bottle to obtain an oily product at the bottom of the cleaning bottle. By the sampling for 10 minutes, 483 g of the oily product was obtained. This product was analyzed by gas chromatography, whereby the product was found to comprise 38% of R123 + R123a, 57% of R122 and 5% of others, as calculated by the area ratios. From the analysis by the NMR method, the ratio of R123 to R123a was at least 99/1.

EXAMPLE 2

By means of the same reactor as used in Example 1, antimony pentachloride was fluorinated. Then, 360 g/hr of HF and 1,600 g/hr of 1,1,2,2-tetrachloro-1-fluoroethane (R121) were supplied at 130°C under 0 K/G. Sampling was conducted for 10 minutes in the same manner as in Example 1 to obtain 183 g of an oily product. By the analysis by gas chromatography, the product was found to comprise 15% of-R121, 51% of R122, 29% of R123 + R123a and 5% of others, as calculated from the area ratios. Further, from the analysis by the NMR, the ratio of R123 to R123a was at least 99/1.

EFFECTS OF THE INVENTION

The present invention is a process whereby R123 can be produced in good yield, as shown by the above Examples and has a feature that formation of isomer R123a as a by-product is very small. Further, the process of the present invention can be applied to a batch method or to a continuous method, and thus is suitable for mass production. Further, the starting materials and catalyst to be used are inexpensive, whereby R123 can be produced at low costs.

**Claims**

1. The process for producing 1,1-dichloro-2,2,2-trifluoroethane (R123), which comprises reacting 1,1,2-trichloro-2,2-difluoroethane (R122) and/or 1,1,2,2-tetrachloro-1-fluoroetane (R121) with HF in a liquid phase in the presence of antimony pentachloride.

2. The process according to Claim 1, wherein HF is used in an amount of from 1 to 3 mols per mol of R122.

3. The process according to Claim 1, wherein HF is used in an amount of from 2 to 6 mols per mol of R121.

4. The process according to any one of Claims 1 to 3, wherein the reaction is conducted at a temperature of from 50 to 150°C.

5. The process according to any one of Claims 1 to 4, wherein the reaction is conducted under a pressure of from 0 to 25 kg/cm$^2$G.

# INTERNATIONAL SEARCH REPORT

International Application No    PCT/JP 89/00803

## I. CLASSIFICATION OF SUBJECT MATTER (if several classification symbols apply, indicate all) ⁴

According to International Patent Classification (IPC) or to both National Classification and IPC

Int, Cl⁴    C07C19/08, 17/20, B01J27/10,
C07B61/00

## II. FIELDS SEARCHED

### Minimum Documentation Searched ⁷

| Classification System | Classification Symbols |
|---|---|
| IPC | C07C19/08, C07C17/10-22, B01J27/10 |

Documentation Searched other than Minimum Documentation
to the Extent that such Documents are Included in the Fields Searched ⁸

## III. DOCUMENTS CONSIDERED TO BE RELEVANT ⁹

| Category * | Citation of Document, ¹¹ with indication, where appropriate, of the relevant passages ¹² | Relevant to Claim No. ¹³ |
|---|---|---|
| Y | JP, A, 52-83402 (Daikin Industries, Ltd.) 12 July 1977 (12.07.77) &DE, A1, 2659046&US, A, 4091043 &GB, A, 1571915 | 1-2, 4-5 |
| Y | JP, A, 50-106904 (Central Glass Co., Ltd.) 22 August 1975 (22.08.75) (Family : none) | 1 |
| Y | JP, B1, 49-3965 (Denki Kagaku Kogyo Kabushiki Kaisha) 29 January 1974 (29.01,74) (Family : none) | 1-2, 4-5 |

* Special categories of cited documents: ¹⁰

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| October 11, 1989 (11.10.89) | October 30, 1989 (30.10.89) |

| International Searching Authority | Signature of Authorized Officer |
|---|---|
| Japanese Patent Office | |

Form PCT/ISA/210 (second sheet) (January 1985)